(19) Europäisches Patentamt
European Patent Office
Office européen des brevets

(11) Veröffentlichungsnummer: **0 158 224 B1**

# (12) EUROPÄISCHE PATENTSCHRIFT

(45) Veröffentlichungstag der Patentschrift: **24.07.91**

(51) Int. Cl.$^5$: **C12Q 1/34**

(21) Anmeldenummer: **85103671.5**

(22) Anmeldetag: **27.03.85**

## (54) Analysenverfahren und Mittel zum Nachweis esterolytischer und/oder proteolytischer Enzyme.

(30) Priorität: **06.04.84 DE 3413120**

(43) Veröffentlichungstag der Anmeldung:
**16.10.85 Patentblatt 85/42**

(45) Bekanntmachung des Hinweises auf die Patenterteilung:
**24.07.91 Patentblatt 91/30**

(84) Benannte Vertragsstaaten:
**DE FR GB IT NL SE**

(56) Entgegenhaltungen:
**EP-A- 0 039 880**
**US-A- 4 212 939**

**CHEMICAL ABSTRACTS, Band 94, Nr. 19, 11. Mai 1981, Seite 279, Zusammenfassung Nr. 152503v, Columbus, Ohio, US; V. RAMESH et al.: "Studies on the kinetics and activation of soluble and immobilized sweet potato beta-amylase", & J. MOL. CATAL. 1981, 10(3), 341-55**

(73) Patentinhaber: **MILES INC.**
**1127 Myrtle Street**
**Elkhart Indiana 46514(US)**

(72) Erfinder: **Schnabel, Eugen, Dr.**
**Schimmelweg 6**
**W-5600 Wuppertal 11(DE)**

(74) Vertreter: **Dänner, Klaus, Dr. et al**
**c/o Bayer AG Konzernverwaltung RP Patentabteilung**
**W-5090 Leverkusen 1 Bayerwerk(DE)**

EP 0 158 224 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 93, Nr. 15, 13. Oktober 1980, Seite 303, Zusammenfassung Nr. 145337v, Columbus, Ohio, US; J.P. BENEDETTO et al.: "Effects of low molecular weight basic proteins on the phosphohydrolase and phosphotransferase activities of microsomal glucose-6-phosphatase in adult monkey hepatocytes", & BIOCHIM. BIOPHYS. ACTA 1980, 614(2), 400-6

CHEMICAL ABSTRACTS, Band 95, Nr. 11, 14. September 1981, Seiten 385-386, Zusammenfassung Nr. 94612n, Columbus, Ohio, US; K.D. MILLER: "Nonenzymic control of prothombrin activation", & ANN. N.Y. ACAD. SCI. 1981, 370(CONTRIB. HEMOSTASIS), 336-47

CHEMICAL ABSTRACTS, Band 91, Nr. 9, 27. August 1979, Seite 263, Zusammenfassung Nr. 70776k, Columbus, Ohio, US; M. YAMAMOTO et al.: "A hepatic soluble cyclic nucleotide-independent protein kinase. Stimulation by basic polypeptides", & J. BIOL. CHEM. 1979, 254(12), 5049-52

## Beschreibung

Die vorliegende Erfindung betrifft Mittel für den analytischen Nachweis von esterolytischen und/oder proteolytischen Enzymen, z.B. in Körperflüssigkeiten, wobei die Ester in geeigneter Weise in Testmitteln, insbesondere Teststreifen, inkorporiert sind. Die erfindungsgemäßen Mittel enthalten neben chromogenen Enzymsubstraten (Aminosäure- oder Peptidestern von geeigneten Phenolen) sowie gegebenenfalls mit den Phenolen unter Farbbildung kuppelnden Diazoniumsalzen noch Polyaminosäuren als Beschleuniger für die enzymatische Spaltung der Aminosäure- bzw. Peptidester. Bevorzugt werden die Mittel für den Nachweis von Leukozyten, insbesondere im Urin, eingesetzt.

In der Diagnostik der Erkrankungen der Nieren und des Urogenitaltraktes hat der Nachweis von Leukozyten in Körperflüssigkeiten, insbesondere im Urin, große Bedeutung. Ursprünglich erfolgte dieser Nachweis durch Auszählen der Leukozyten im nicht zentrifugierten Harn oder im Harnsediment. Mit beiden Methoden können nur intakte Leukozyten erfaßt werden. Es ist jedoch bekannt, daß die Geschwindigkeit der Leukozyten-Lyse je nach Harnmilieu starken Schwankungen unterworfen ist; so beträgt z.B. in stark alkalischen Harnen die Leukozyten-Halbwertszeit nur 60 Minuten. Dies führt dazu, daß zu niedrige Leukozytenzahlen festgestellt werden. Von diesem Lyse-Fehler abgesehen, liefert die quantitative mikroskopische Bestimmung der Leukozyten im nicht zentrifugierten, homogenisierten Harn in der Zählkammer sehr genaue Werte. Trotzdem wird diese Methode in der Praxis nur selten angewandt, da sie mühevoll und zeitraubend ist und geschultes Personal voraussetzt.

Das in der medizinischen Praxis bevorzugte Verfahren für die Leukozytenbestimmungen im Harn war daher die sogenannte Gesichtsfeldmethode im Harnsediment. Hierzu mußte zunächst die Probe (Sediment) durch Zentrifugieren gewonnen werden. Dabei wurden jedoch auch andere Bestandteile des Harnes angereichert, die - wie z.B. Salze und Epithelzellen - die mikroskopische Auszählung der Leukozyten beträchtlich erschweren. Schwankender Sedimentgehalt, Inhomogenitäten des Sediments sowie unterschiedliche optische Ausstattung der Mikroskope führten zu relativ großen Fehlern (bis zu mehreren hundert Prozent) bei der Angabe der Leukozytenzahl.

Um diese Schwierigkeiten zu vermeiden, wurde bereits vielfach versucht, als Nachweisprinzip für Leukozyten in verschiedenen Körperflüssigkeiten enzymatische Reaktionen heranzuziehen, da die Leukozyten ein breitgefächertes Enzymspektrum besitzen.

So sind z.B. aus den deutschen Offenlegungsschriften 2 826 965 und 2 836 644 Mittel zum Nachweis von Leukozyten in Körperflüssigkeiten bekannt, bei denen die in Leukozyten vorhandene esterolytische und/oder proteolytische Aktivität für analytische Zwecke ausgenutzt wird. Als Substrate für die Leukozyten-Esterasen und/oder -Proteasen werden dabei Sulfonphthaleinester bzw. Azo-Farbstoffester verwendet. Die bei der enzymatischen Umsetzung freigesetzten Farbstoffe werden dann nach bekannten Methoden bestimmt. Die in diesen Publikationen beschriebenen Mittel sind jedoch für praktische Zwecke noch zu unempfindlich, da sie bei niedrigen Leukozyten-Konzentrationen zu lange Reaktionszeiten aufweisen.

Verschiedene Methoden für den Nachweis von Proteasen und Esterasen sind auch aus der histo- und cytochemischen Enzymologie bekannt <vgl. beispielsweise A.G.E. Pearse, Histochemistry, Theoretical and Applied, 3. Ed., Churchill Livingstone, Edinburgh-London-New York 1968). Zum Nachweis werden dabei im allgemeinen farblose oder schwach gefärbte Ester eingesetzt, die durch die Enzyme in eine farblose Säure und eine ebenfalls farblose Alkohol-(Phenol)-Komponente gespalten werden. Die Phenolkomponente wird dann in einer Folgereaktion zu farbigen Produkten umgesetzt, z.B. durch Kupplung mit Diazoniumsalzen oder durch Oxidation. F. Schmalzl und H. Braunsteiner beschreiben zum Beispiel in Klin. Wschr. 46, 642 (1968) einen spezifischen cytochemischen Leukozytenesterasenachweis mit Naphthol-AS-D-Chloracetat als Substrat und einem Diazoniumsalz, das mit dem freiwerdenden Naphthol eine farbige Azo-Verbindung bildet.

Für den schnellen und einfachen Nachweis von Leukozyten in Körperflüssigkeiten, wie z.B. im Harn, erwiesen sich Zweikomponenten-Systeme dieser Art jedoch als nicht geeignet, da sie viel zu unempfindlich sind: Proben mit 5000 Leukozyten/$\mu$l zeigen noch keine Reaktion.

In GB-A 1 128 371 und EP-A 12 957 wird die Verwendung von Indoxyl- und Thioindoxylestern als chromogenen Substraten für den Nachweis von hydrolytischen Enzymen in Körperflüssigkeiten beschrieben. Bei der enzymatischen Spaltung des Substrats entsteht freies Indoxyl, welches anschließend zum leicht nachweisbaren blauen Farbstoff Indigo oxidiert wird. Ein handelsüblicher Test auf Basis von EP-A 12 957 besteht aus einem Streifen Filterpapier, welches mit dem N-Tosyl-L-alanin-L-indoxylester imprägniert ist. Beim Eintauchen in eine Leukozyten enthaltende Urinprobe färbt sich der Teststreifen blau. Ein wesentlicher Nachteil dieses Produkts ist jedoch die lange Wartezeit (ca. 15 Minuten), bis die Endfärbung erreicht ist und der Test ausgewertet werden kann.

In EP-A 14 929 werden verschiedenartige Beschleuniger (Pyridinderivate; Imidazolderivate; Alkohole;

Metallkomplexe) für die enzymatische Spaltungsreaktion beschrieben. Nachteilig bleiben jedoch die relativ lange Zeit bis zur vollständigen Oxidation des Indoxyls und die geringe Empfindlichkeit des Tests (Nachweisgrenze: einige Tausend Leukozyten/$\mu$l). Gleiches trifft auch für die Verwendung der Ester von Leuko-Indoanilinen als Substrate für Leukozyten-Enzyme gemäß EP-A 34 323 zu.

EP-A 39 880 stellt eine Kombination der Substrate gemäß EP-A 12 957 bzw. 14 929 mit dem weiter oben diskutierten Nachweisprinzip der Kupplung mit Diazoniumsalzen dar. Es gelingt auf diese Weise zwar, die Erfassungsgrenzen für Leukozyten deutlich zu senken, jedoch wird die für die Praxisanwendung gewünschte Nachweisempfindlichkeit von 15 - 20 Leukozyten/$\mu$l noch nicht erreicht.

Aufgabe der vorliegenden Erfindung war es daher, neue Beschleuniger für esterspaltende Enzyme aufzufinden, die infolge einer Beschleunigung der enzymatischen Spaltung der Substrate durch die Leukozytenenzyme einen empfindlicheren und rascheren Nachweis der Leukozyten im Harn ermöglichen. Diese Aufgabe wird durch den Zusatz von Polyaminosäuren zu dem Reagenssystem gelöst. Die Polyaminosäuren übertreffen überraschenderweise die in EP-A 14929 beschriebenen Beschleuniger (Pyridinderivate, Imidazolderivate, Metallkomplexe und Alkohole) in ihrer die Leukozytenenzyme beschleunigenden Wirkung. Sie sind zudem auch als Detergentien brauchbar, so daß sich eine gleichzeitige Zugabe von Detergentien zu den in der Praxis verwendeten Reagenssystemen (z. B. Reaktionslösungen bzw. Formulierungen für die Beschichtung von Teststreifen) erübrigt.

Gegenstand der Erfindung sind Mittel zum Nachweis esterolytischer und/oder proteolytischer Enzyme, enthaltend

(a) einen Aminosäure- oder Peptidester eines Phenols als chromogenes Enzymsubstrat,
(b) eine die enzymatische Spaltung der Aminosäure- bzw. Peptidesterbindung von Komponente (a) beschleunigende Substanz, gegebenenfalls
(c) ein Diazoniumsalz, gegebenenfalls
(d) einen Puffer, sowie gegebenenfalls
(e) ein Trägermaterial und/oder übliche Zusatzstoffe,

dadurch gekennzeichnet, daß Komponente (b) eine vorzugsweise basische Gruppen aufweisende Polyaminosäure mit einem Molekulargewicht (Zahlenmittel) zwischen 1000 und 2 000 000 ist.

Gegenstand der Erfindung ist schließlich auch ein Verfahren zum Nachweis von esterolytischen und/oder proteolytischen Enzymen in flüssigen Proben, insbesondere Körperflüssigkeiten, welches dadurch gekennzeichnet ist, daß man die Probe mit dem erfindungsgemäßen Mittel in Kontakt bringt und die auftretende Farbreaktion bestimmt.

Als erfindungsgemäß einzusetzende Beschleuniger kommen sowohl Polyaminosäuren in Betracht, die aus nur einer einzigen Aminosäure aufgebaut sind (Homopolyaminosäuren) als auch Polykondensate aus zwei oder mehreren verschiedenen Aminosäuren in Form von Co-polyaminosäuren mit einer randomisierten Sequenz der konstituierenden Aminosäure oder in Form von Sequenzpolymeren. Sequenzpolymere werden erhalten bei der Polykondensation von Peptiden bzw. ihren Derivaten und besitzen eine definierte, sich wiederholende Aminosäuresequenz. Für die beschleunigende Wirkung der Polyaminosäuren ist es von besonderem Vorteil, wenn mindestens eine der für die Polymerisation bzw. Co-polymerisation eingesetzen Aminosäuren eine basische Gruppe trägt, z. B. eine Aminogruppe oder Guanidinogruppe.

Vorzugsweise sind die erfindungsgemäß zu verwendenden Polyminosäuren aus gleichen oder verschiedenen Monomeren der allgemeinen Formel

$$H_2N-\underset{\displaystyle R}{\underset{|}{CH}}-COOH \qquad (I)$$

aufgebaut, in welcher R für Wasserstoff oder einen gegebenenfalls verzweigten Alkyl-, Cycloalkyl- oder Aralkylrest mit 1 bis 15 C-Atomen, bevorzugt 1 bis 9 C-Atomen, steht, welcher gegebenenfalls durch eine oder zwei, insbesondere eine, Hydroxy-, Mercapto-, Carboxyl-, Amino- oder Guanidinogruppen substituiert ist.

Bevorzugt enthalten die erfindungsgemäß als Beschleuniger zu verwendenden Polyaminosäuren 5 bis 100 Mol-%, insbesondere 10 bis 100 Mol-%, an Monomerbausteinen mit einer basischen Gruppe. Als solche Monomerbausteine besonders bevorzugt sind Aminosäuren der allgemeinen Formel (I), welche im Rest R eine Amino- oder Guanidinogruppe aufweisen. Beispiele für solche basischen Aminosäuren sind insbesondere Arginin, Lysin und Ornithin, die sowohl racemisch vorliegen können als auch in der L- oder D-Form. Darüber hinaus können die Polyaminosäuren und die Sequenzpolymeren als Bausteine auch nicht in

natürlichen Proteinen vorkommende basische Aminosäuren enthalten. Beispielhaft seien hier α, γ-Diaminobuttersäure, α, β-Diaminopropionsäure und die Diaminopimelinsäurenerwähnt.

Die erfindungsgemäß als Beschleuniger der enzymatischen Spaltung zu verwendenden Polyaminosäuren sind literaturbekannt und kommerziell verfügbar, bzw. sind sie nach an sich bekannten Verfahren herstellbar (siehe z. B.

E. Katchalski und M. Sela in: Advances of Protein Chemistry 13, 243-492 (1958); C.L. Anfinsen, M.L. Anson, J.T. Edsall und K. Bailey (Hrsg.) in Academic Press Inc. Publishers, New York.

Die mittleren Molmassen (Zahlenmittel) der Polyaminosäuren sollen zwischen 1000 und 2 000 000 liegen, vorzugsweise werden Polyaminosäuren mit mittleren Molekulargewichten von 5 000 bis 500 000, besonders bevorzugt 10 000 bis 300 000, verwendet.

Die Polyaminosäuren werden im homogenen Flüssigtest vorzugsweise in Konzentrationen von 0.0001 Gew.-% bis 1 Gew.-% eingesetzt, besonders bevorzugt liegen die Konzentrationen im Bereich von 0.001 - 0.01 Gew.-%. Bei der unten beschriebenen Herstellung von Testvorrichtungen werden sie, bezogen auf die Imprägnierlösung, in Konzentrationen von 0.05 Gew.-% bis 10 Gew.-%, vorzugsweise von 1 bis 5 Gew.-%, eingesetzt. Die erfindungsgemäß zu verwendenden Polyaminosäuren beschleunigen die enzymatische Spaltung der in den EP-A 7407, 8428, 12 957, 14 929, 34 323 und 39 880 beschriebenen Substrate durch die Leukozytenenzyme ebenso wie die Spaltung der bereits früher beschriebenen Substrate [G.Gomori, J. Histochem. Cytochem. 6 469 (1953); H. Löffler, Klin. Wochenschr. 39, 1120 (1961); L. Visser und E. Blout, Fed.-Proc. 28, 407 (1969) sowie Biochim. Biophys. Acta 268, 257 (1972) und F. Sweetman und L. Ornstein, J. Histochem. Cytochem. 23, 327 (1974)].

Zu den bevorzugten chromogenen Substraten in den erfindungsgemäßen Mitteln gehören auch die in einer parallelen Anmeldung beschriebenen Verbindungen der allgemeinen Formel (II)

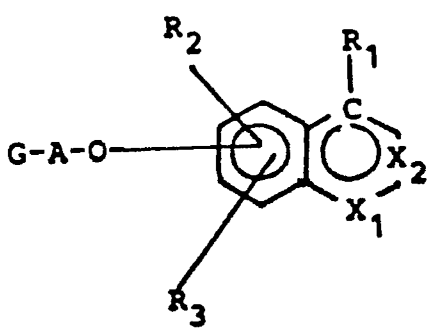

(II)

in welcher

$X_1$ und $X_2$ gleich oder verschieden sind und Stickstoff oder Schwefel bedeuten, mit der Maßgabe, daß $X_1$ und $X_2$ nicht gleichzeitig für Schwefel stehen;

$R_1$ für Wasserstoff oder eine gegebenenfalls verzweigte Alkylgruppe mit 1 bis 6 C-Atomen steht, die gegebenenfalls durch Halogen oder Hydroxy substituiert sein kann;

$R_2$ und $R_3$ gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkylgruppen, $C_1$-$C_6$-Alkoxygruppen, Halogen, Cyano, Nitro, Trifluormethyl, $SO_3H$,$C_1$-$C_6$-Acylgruppen oder $C_1$-$C_6$-Dialkylaminogruppen substituiert sein können, oder $R_2$ und $R_3$ gemeinsam einen annellierten aromatischen Ring, vorzugsweise einen Benzolring, bilden, welcher seinerseits mit einem oder zwei Resten $R_2$ substituiert sein kann;

A einen Aminosäure- oder Peptidrest bedeutet und

G Wasserstoff oder vorzugsweise eine in der Peptidchemie übliche oder davon abgeleitete Stickstoffschutzgruppe darstellt.

Bevorzugte Verbindungen der allgemeinen Formel (II) sind solche, bei denen $X_1$ für Schwefel und $X_2$ für Stickstoff stehen. Bevorzugt sind weiterhin Verbindungen der Formel (II), in denen $R_1$ für Wasserstoff steht und solche, bei denen $R_2$ und $R_3$, die gleich oder verschieden sind, für Wasserstoff, $C_1$-$C_2$-Alkyl, $C_1$-$C_2$-Alkoxy, Halogen, $C_1$-$C_4$-Dialkylaminogruppen oder Benzolreste stehen.

Besonders bevorzugt steht bei den Verbindungen von Formel (II) der Esterrest in 5-Position.

Weitere erfindungsgemäß bevorzugte chromogene Substrate sind die ebenfalls in einer parallelen

Anmeldung beschriebenen Verbindungen der allgemeinen Formel (III)

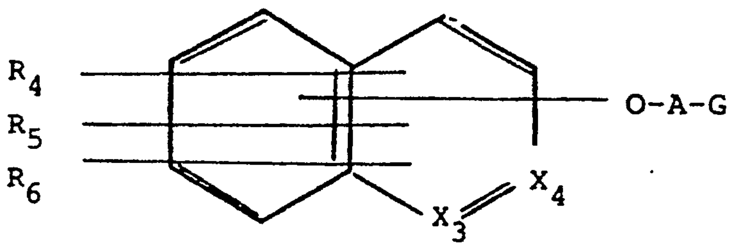

in welcher

| | |
|---|---|
| $X_3$ und $X_4$ | für N oder CH stehen mit der Maßgabe, daß jeweils entweder $X_3$ oder $X_4$ für N steht, |
| $R_4$, $R_5$ und $R_6$ | gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkylgruppen, $C_1$-$C_6$-Alkoxygruppen, Halogen, Cyano, Nitro, Trifluormethyl, $SO_3H$, $C_1$-$C_6$-Acylgruppen oder $C_1$-$C_6$-Dialkylaminogruppen substituiert sein können, oder $R_5$ und $R_6$ gemeinsam einen annellierten aromatischen Ring, vorzugsweise einen Benzolring, bilden, welcher seinerseits mit einem oder zwei Resten $R_4$ substituiert sein kann, |

und A und G die oben bei Formel (II) angegebene Bedeutung haben.

In den Verbindungen gemäß allgemeiner Formel (III) steht vorzugsweise $X_3$ für CH und $X_4$ für Stickstoff. Bevorzugt bedeuten $R_4$, $R_5$ und $R_6$, die gleich oder verschieden sein können, für Wasserstoff, $C_1$-$C_4$-Alkyl, $C_1$-$C_4$-Alkoxy, Acylamino (wobei der Säurerest aliphatisch oder aromatisch mit 1 bis 6 C-Atomen sein kann), $C_1$-$C_4$-Dialkylamino, Nitro, Cyano, Halogen sowie Aryl, das gegebenenfalls substituiert ist durch $C_1$-$C_4$-Alkyl, $C_1$-$C_4$-Alkoxy oder Halogen.

Besonders bevorzugt sind $R_4$, $R_5$ und $R_6$ Wasserstoff, $C_1$-$C_4$-Alkyl, Phenyl oder Halogen oder $R_5$ und $R_6$ bilden zusammen einen annellierten Benzolring.

Als chromogene Substrate für die erfindungsgemäßen Mittel eignen sich darüber hinaus auch Verbindungen der allgemeinen Formel

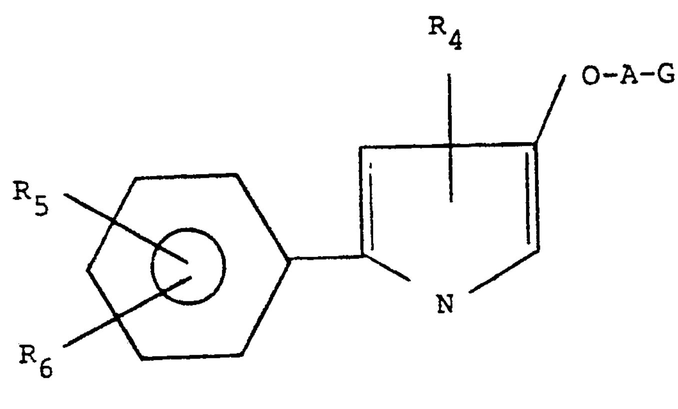

(IV)

wobei $R_4$, $R_5$, $R_6$, A und G die oben bei Formel (III) angegebene Bedeutung haben.

In den allgemeinen Formeln III), (III) und (IV) steht G - A - bevorzugt für einen Rest der allgemeinen Formel

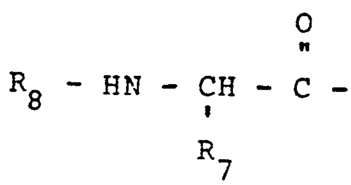

in welcher

$R_7$ für Wasserstoff oder einen gegebenenfalls verzweigten Alkyl-, Cycloalkyl- oder Arylrest mit 1-15 C-Atomen, bevorzugt 1-9 C-Atomen, steht, welcher gegebenenfalls durch eine Hydroxy-, eine Mercapto- oder eine Carboxylgruppe substituiert ist, und

$R_8$ Wasserstoff oder vorzugsweise -CO-Alkyl, -CO-Aralkyl, -CO-Aryl, $-SO_2$-Alkyl oder $-SO_2$-Aryl darstellt, wobei Alkylreste geradkettig oder verzweigt mit 1-9 C-Atomen, bevorzugt 1-6 C-Atomen sind und Arylreste bevorzugt Benzolringe, die gegebenfalls durch $C_1$-$C_4$-Alkylgruppen, $C_1$-$C_4$-Alkoxygruppen oder Halogen substituiert sind, darstellen.

Besonders bevorzugt steht G-A- für einen mit einer üblichen Stickstoffschutzgruppe versehenen Rest einer natürlichen Aminosäure oder eines Peptids aus 2 bis 8 solcher Aminosäuren.

Die Aminosäurereste können dabei in ihrer L- oder D-Form oder auch in ihrer racemischen Form vorliegen. Besonders bevorzugt sind die Reste von Glycin, Alanin, Valin, Leucin, Isoleucin, Phenylalanin und Tyrosin, wobei jeweils die L-Form besonders bevorzugt ist. Gegebenenfalls vorhandene freie Hydroxygruppen können acyliert, vorzugsweise acetyliert, sein.

Unter einem Peptidrest in der Definition von A sind z.B. Di-, Tri-, Tetra- und Pentapeptide, vorzugsweise Di- und Tripeptide, zu verstehen, wobei als Aminosäure-Komponenten vorzugsweise die oben erwähnten Aminosäuren in Betracht kommen.

Die Substrate der allgemeinen Formeln (II), (III) und (IV) werden erhalten, indem man die entsprechenden Phenole mit Aminosäuren bzw. Peptiden der allgemeinen Formel

G-A-OH

in welcher G und A die oben angegebenen Bedeutung haben, bzw. geeigneten reaktiven Derivaten davon nach in der Peptidchemie üblichen Methoden umsetzt.

Geeignete reaktive Derivate sind z. B. die Säurechloride und die bei der Peptidsynthese üblicherweise verwendeten Mischanhydride, z. B. mit Chlorameisensäureethylester oder Aktivester wie z. B. Pentachlorphenylester oder N-Hydroxybenztriazolester.

Die erfindungsgemäßen Mittel enthalten vorzugsweise als mit den (bei der enzymatischen Spaltung freigesetzten) Phenolen reagierende Farbbildner Diazoniumsalze der allgemeinen Formel

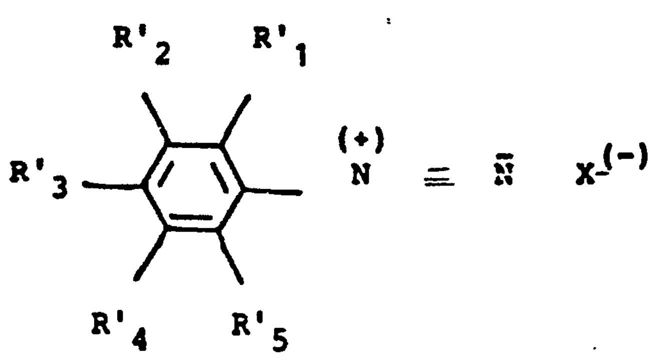
(V)

in der

$R'_1$ eine niedere Alkyl-, eine niedere Alkoxy-, eine niedere Alkylmercapto-, eine Hydroxy-, Nitro-, Cyano-, Trifluormethyl-, $C_1$-$C_8$-Alkylsulfonamido-, Arylsulfonamido-, $C_1$-$C_8$-Alkylsulfon-, Arylsulfon-, Sulfonsäure-, Carbonsäure-, eine N-Morpholino-, eine N-Thiomorpholino-, eine N-Pyrrolidino-, eine gegebenenfalls N'-alkylierte N-Piperazino-, eine N-Piperidino-Gruppe, Halogen oder Wasserstoff,

$R'_3$ eine niedere Alkyl-, eine niedere Alkoxy-, eine Aryloxy-, eine niedere Alkylmercapto-,

Alkylamino-, Dialkylamino-, eine Hydroxy-, Nitro-, Cyano-, $C_1$-$C_8$-Alkylsulfonamido-, Arylsulfonamido, $C_1$-$C_8$-Alkylsulfon-, Arylsulfon-, Sulfonsäure-, Carbonsäure, eine N-Morpholino-, N-Thiomorpholino-, N-Pyrrolidino-, eine gegebenenfalls N'-alkylierte N-Piperazino-, N-Piperidino-, Phenylamino-, eine gegebenenfalls mit einem niederen Alkyl- oder niederen Alkoxy-Rest substituierte Phenyl-Gruppe, Halogen oder Wasserstoff,

R'2, R'4, R'5, die gleich oder verschieden sein können, jeweils eine niedere Alkyl-, eine niedere Alkoxy-, Nitro-, $C_1$-$C_8$-Alkylsulfonamido-, Arylsulfonamido-, $C_1$-$C_8$-Alkylsulfon-, Arylsulfon-, Sulfonsäure-, Carbonsäure-, eine niedere Alkylmercapto-Gruppe, Halogen oder Wasserstoff und

X ein stabilisierendes Anion

bedeuten, wobei jeweils 2 benachbarte Reste $R'_1$ bis $R'_5$ zu einem gegebenenfalls durch Halogen; eine $C_1$-$C_6$-Alkyl-, eine $C_1$-$C_6$-Alkoxy-, eine Nitro-, Sulfonsäure- oder Carbonsäuregruppe substituierten Benzolring ringgeschlossen sein können, so daß ein Diazoniumsalz der Naphthalinreihe entsteht.

Vorzugsweise stehen in der allgemeinen Formel (V)

$R'_1$ für $C_1$- bis $C_4$-Alkyl, $C_1$-$C_4$-Alkoxy, Hydroxy, Nitro, Halogen oder Wasserstoff

$R'_3$ für eine $C_1$-bis $C_4$-Alkyl-, $C_1$-$C_4$-Alkoxy-, Aryloxy-, $C_1$-$C_4$-Alkylamino-, $C_1$-$C_4$-Dialkylamino-, Nitro-, $C_1$-$C_4$-Alkyl-Sulfonamido-, Arylsulfonamido-, $C_1$-$C_4$-Alkylsulfon-, Arylsulfon-, N-Morpholino-, N-Pyrrolidino-, phenylamino- oder Sulfonsäuregruppe oder Wasserstoff und

$R'_2$, $R'_4$, $R'_5$, die gleich oder verschieden sein können, für $C_1$- bis $C_4$-Alkyl-, $C_1$- bis $C_4$-Alkoxy-, $C_1$-bis $C_4$-Alkylamino-,$C_1$-bis $C_4$-Dialkylamino-, Nitro-, $C_1$-bis $C_4$-Alkylsulfonamido-, Arylsulfonamido- oder sulfonsäuregruppen, Halogen oder Wasserstoff.

Jeweils 2 benachbarte Reste $R'_1$ bis $R'_5$ können dabei gegebenenfalls zu einem gegebenenfalls durch Halogen, eine $C_1$- bis $C_4$-Alkyl-, $C_1$- bis $C_4$-Alkoxy-, eine Nitro-, oder Sulfonsäuregruppe substituierten Benzolring ringgeschlossen sein.

Im Rahmen der Formel (V) steht Aryl jeweils für einen gegebenenfalls durch Halogen, eine $C_1$-$C_4$-Alkyl- oder $C_1$-$C_4$-Alkoxygruppe substituierten aromatischen Rest mit 6 bis 12 C-Atomen, vorzugsweise 6 C-Atomen.

Die Diazoniumsalze der allgemeinen Formel (V) sind an sich bekannt oder können nach an sich bekannten Methoden synthetisiert werden (Houben-Weyl, Methoden der organischen Chemie, Band X/3).

Vorzugsweise enthalten die erfindungsgemäßen Mittel zum Nachweis proteolytischer Enzyme und insbesondere der Leukozyten-Enzyme ein geeignetes Puffersystem. Hierfür kommen z. B. Phosphat-, Borat-, Carbonat/Hydrogencarbonat-, Carbonat-, Barbiturat-, Tris-(hydroxymethyl)-aminomethan-(=Tris)-, 2 Amino-2-methyl-propandiol-1,3-(=Amediol)- oder Aminosäure-Puffer in Frage, wobei in der Regel pH-Wert und Kapazität so gewählt werden, daß sich in der Meßlösung bzw. auf dem Teststreifen ein pH-Wert von 6 - 10, vorzugsweise von 7 - 9, einstellt.

In manchen Fällen kann es vorteilhaft sein, in den erfindungsgemäßen Mitteln neben den beschleunigend wirkenden Polyaminosäuren auch Detergentien mitzuverwenden.

Diese Detergentien bewirken einmal eine Desintegration der in der Testlösung vorhandenen Leukozyten und setzen so die Enzyme frei, zum anderen wirken sie lösungsvermittelnd für die Substrate und die bei der Spaltung entstehenden Substanzen und gegebenenfalls farbvertiefend. Als Detergentien kommen sowohl kationenaktive als auch anionenaktive Detergentien aber auch amphotere und nichtionogene Detergentien in Frage.

Als Beispiele hierfür seien Benzyl-dimethyl-tetradecylammoniumchlorid, Na-Dodecylsulfat, Zephirol, Polyvinylpyrrolidon und Heparinoid genannt, gegebenenfalls können auch Gemische von zwei oder mehreren der oben erwähnten Detergentien eingesetzt werden. Da die erfindungsgemäß als Aktivatoren eingesetzten Polyaminosäuren auch Detergenswirkung haben, kann im Prinzip jedoch auf die Zugabe zusätzlicher Detergentien verzichtet werden.

Besonders vorteilhaft ist die Verwendung der obigen Detergentien bei der Bestimmung der Leukozyten mittels auf einer festen Phase (z. B. einem Teststreifen) fixierten Reagentien, da hierdurch eine homogenere Farbverteilung und eine intensivere Färbung erzielt werden können.

Vorzugsweise sind bei den erfindungsgemäßen Mitteln die verschiedenen oben beschriebenen Reagenzien in einem inerten Trägermaterial der an sich bekannten Art inkorporiert, wobei als Trägermatrix besonders bevorzugt poröse Materialien wie insbesondere Filterpapier, aber auch Kunststoffmembranen, Glasfasermatten (US-PS 3 846 247), poröse Keramikstreifen, Kunstfaservliese, schwammartige Materialien (US-PS 3 552 928), Filz, Textilien, Holz, Cellulose oder auch Silicagel in Frage kommen.

Die genannten Trägermaterialien werden zu diesem Zweck mit einer Lösung der oben beschriebenen Reagentien in einem geeigneten leicht entfernbaren Lösungsmittel, z.B. Wasser, Methanol, Ethanol, Aceton, DMF oder DMSO imprägniert. Vorzugsweise geschieht dies in zwei getrennten Schritten: Zunächst wird mit einer wäßrigen Lösung imprägniert, die den Puffer und andere wasserlösliche Zusatzstoffe enthält. Danach

wird mit einer Lösung der chromogenen Enzym-Substrate der allgemeinen Formel (V) und Aktivatoren imprägniert. Die Imprägnierung kann jedoch auch in einer anderen Reihenfolge bzw. mit einer anderen Zusammensetzung der beiden Imprägnierlösungen durchgeführt werden. Vorzugsweise enthält die Imprägnierlösung oder die zu untersuchende Flüssigkeit das chromogene Substrat und das Diazoniumsalz jeweils in einer Konzentration von $10^{-4}$ bis $10^{-1}$ mol/l, insbesondere $10^{-3}$ bis $10^{-2}$ mol/l, und die Polyaminosäure in einer Konzentration von 0,05 Gew.-% bis 10 Gew.-%, insbesondere von 1 Gew.-% bis 5 Gew.-%.

Bei Verwendung von Filterpapier als Matrix können die fertigen Testpapiere als solche verwendet werden oder in an sich bekannter Weise an Griffen angeklebt oder vorzugsweise zwischen Kunststoffen und feinmaschigen Netzwerken z.B. gemäß DE-OS 2 118 455 eingesiegelt werden.

Zur Herstellung filmbeschichteter Teststreifen werden vorzugsweise sämtliche Reagenzien in die Lösung oder Dispersion einer filmbildenden Substanz, wie z.B. Polyvinylester oder Polyamid, eingetragen und homogen vermischt. Das Gemisch wird in dünner Schicht auf einen Kunststoffträger gestrichen und getrocknet. Die so hergestellten filmbeschichteten Teststreifen werden nach dem Trocknen geschnitten und können als solche verwendet oder in an sich bekannter Weise an Griffen angeklebt werden oder z.B. zwischen Kunststoffen und feinmaschigen Netzwerken gemäß DE-OS 2 118 455 eingesiegelt werden.

Ein erfindungsgemäßes diagnostisches Mittel zum Nachweis esterolytischer und/oder proteolytischer Enzyme, insbesondere der Leukozyten-Enzyme, in Form von Pulvermischungen oder Reagenztabletten läßt sich herstellen, indem die oben angeführten Bestandteile des Testmittels mit üblichen galenischen Zusatzstoffen versetzt und granuliert werden. Zusatzstoffe dieser Art sind z.B. Kohlenhydrate, wie z.B. Mono-, Oligo- oder Polysaccharide, oder zuckeralkohole, wie z.B. Mannit, Sorbit oder Xylit, oder andere lösliche inerte Verbindungen, wie Polyethylenglykole oder Polyvinylpyrrolidon. Die Pulvermischungen oder Reagenztabletten weisen z.B. ein Endgewicht von ungefähr 50 - 200 mg, vorzugsweise 50 - 80 mg, auf.

Zur Herstellung von Lyophilisaten im Gesamtgewicht von jeweils etwa 5 - 20 mg, vorzugsweise etwa 10 mg, wird eine Lösung gefriergetrocknet, die neben sämtlichen für den Test benötigten Reagenzien übliche Gerüstbildner, wie z.B. Polyvinylpyrrolidon, und evtl. weitere Füllstoffe, wie z.B. Mannit, Sorbit oder Xylit, enthält.

Ein erfindungsgemäßes diagnostisches Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z.B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Die so hergestellten diagnostischen Mittel ermöglichen es, nach Eintauchen in die zu untersuchende oder nach Zugabe zur betreffenden Körperflüssigkeit die Anwesenheit esterolytischer und/oder proteolytischer Enzyme, insbesondere der Leukozyten-Enzyme, rasch und einfach über eine Farbbildung nachzuweisen, die visuell oder photometrisch, z.B. remissions-photometrisch oder in der Küvette, gemessen werden kann. Da die Aktivität der Leukozyten-Enzyme pro Zelle als eine im wesentlichen konstante Größe angesehen werden kann, läßt sich aus der Intensität der Farbbildung die Leukozytenkonzentration der untersuchten Körperflüssigkeit ermitteln. Dabei werden mit dem erfindungsgemäßen diagnostischen Mittel sowohl intakte als auch lysierte Leukozyten erfaßt, da die Aktivität der Leukozyten-Enzyme auch nach der Lyse der Leukozyten voll erhalten bleibt. Ein Lysefehler tritt folglich nicht auf.

Die nachfolgenden Beispiele dienen zur Erläuterung der vorliegenden Erfindung. Wenn nicht anders vermerkt, sind Mengenangaben als Gewichtsteile oder Gewichtsprozente zu verstehen.

Allgemeine Vorgangsweise

Zu 2,25 ml des betreffenden Puffers fügte man je nach Substrat 50-250 $\mu$l N-Methylpyrrolidon als Lösungsvermittler und ergänzte das Volumen der Lösung mit Puffer auf 2,5 ml. Dann wurden 5 $\mu$l einer Lösung von 5 - 100 mg Polyaminosäure in 1 ml Wasser oder N-Methylpyrrolidon und 5 $\mu$l einer Lösung von 2 mg Na-Dodecylsulfonat (SDS) bzw. 4 mg der anderen Detergentien in 1 ml Wasser oder N-Methylpyrrolidon zugefügt. Nach gutem Durchmischen wurden 5 $\mu$l einer $10^{-1}$ molaren Substratlösung in N-Methylpyrrolidon bzw. dem angegebenen Lösungsmittel zugefügt und nach der Zugabe der Leukozytensuspension der Extinktionsanstieg bei der angegebenen Wellenlänge kontinuierlich verfolgt. In einem Paralellansatz ohne Leukozytenzusatz wird die durch Spontanverseifung bedingte Extinktionszunahme bestimmt.

Für die Bestimmung der Umsetzungsgeschwindigkeit wird der in der Enzymreaktion erhaltene Extinktionsanstieg um den für die Spontanhydrolyse gefundenen Wert vermindert. Absolutwerte für die Substratspaltung (Mol/min.) können aus den Extinktionsdifferenzen mit Hilfe der molaren Extinktionskoeffizienten errechnet werden.

Beispiel 1

Einfluß des Zusatzes von Polyaminosäuren auf die Geschwindigkeit der Spaltung von Tosyl-L-alanin-indoxylester durch Leukozyten in 0.1 M Tris-(hydroxymethyl)-aminomethan-Puffer pH 8.8 (Zugabe von 50 μl N-Methylpyrrolidon je Testansatz). Die Spaltungsgeschwindigkeiten wurden durch kontinuierliche Messung des Extinktionsanstiegs bei 360 nm bestimmt.

Tabelle 1

| Aktivator | μg/Test | relative Spaltungsgeschwindigkeit |
|---|---|---|
| Decanol | 125 | 1 |
| Poly-L-Arg (40000) | " | 1.75 |
| Poly-D-Lys (100000) | " | 2.05 |
| Poly-DL-Lys (37000) | " | 3.10 |
| Poly-L-Lys (14000) | " | 1.82 |
| " (22000) | " | 1.94 |
| " (55000) | " | 2.37 |
| " (100000) | 50 | 2.10 |
| " (100000) | 125 | 2.35 |
| " (100000) | 250 | 2.50 |
| " (260000) | 125 | 1.82 |
| " (520000) | 50 | 1.75 |
| " (520000) | 125 | 2.20 |
| " (520000) | 250 | 2.10 |
| Poly-(L-Lys, L-Ala) = 2:1 (32000) | 50 | 1.75 |
| " (32000) | 125 | 1.90 |
| " (32000) | 250 | 2.00 |
| Poly-L-Orn (30000) | 125 | 1.80 : 2.90 |

In den Klammern sind die mittleren Molekulargewichte (Zahlenmittel) der Polyaminosäuren angegeben. Als Detergens wurde jeweils SDS in einer Menge von 10 μg pro Testansatz mitverwendet.

Beispiel 2

Beschleunigung der Spaltung von 5-[N-Tosyl-L-alanyloxy]-1,2-benzisothiazol durch Leukozyten bei Zusatz von Polyaminosäuren in 0.1 M Tris-(hydroxymethyl)-aminomethan-Puffer pH 8.4 in Gegenwart von 100 μl N-Methylpyrrolidon je Testansatz. Die Spaltungsgeschwindigkeiten wurden durch kontinuierliche

Messung des Extinktionsanstiegs bei 325 nm bestimmt.

Tabelle 2

| Aktivator | | µg/Test | relative Spaltungsgeschwindigkeit |
|---|---|---|---|
| Decanol | | 125 | 1.0 |
| Poly-L-Arg | (40000) | 25 | 2.4 |
| " | | 30 | 3.6 |
| " | | 125 | 3.7 |
| " | | 250 | 3.4 |
| Poly-L-Lys | (14000) | 125 | 7.8 |
| Poly-L-Orn | (30000) | 125 | 7.0 |
| Poly-L-Arg | (40000) | 25 | 3.0 |
| " | | 50 | 2.8 |
| " | | 125 | 4.0 |
| " | | 250 | 3.3 |
| " | | 375 | 3.4 |
| " | | 500 | 3.2 |
| Poly-L-Lys | (14000) | 500 | 5.6 |
| Poly-L-Orn | (30000) | 500 | 4.3 |
| Poly-L-Arg | (40000) | 500 | 2.95; 2.4 |
| Poly-L-Lys | (14000) | 500 | 3.4 |
| Poly-L-Orn | (30000) | 500 | 3.0 |

In den Klammern sind die mittleren Molekulargewichte (Zahlenmittel) der Polymeren angegeben.

Als Detergens wurde SDS, bei den letzten drei Versuchen von Tabelle 2 Zephirol (als ca. 50 %ige wäßrige Lösung), jeweils in einer Menge von 10 µg pro Testansatz, zugegeben.

Beispiel 3

Beschleunigung der Spaltung von Tosyl-L-alanin-3-hydroxy-5-phenylpyrrolester durch Leukozyten bei Zusatz von Polyaminosäuren in 0.1 M Tris-(hydroxymethyl)-aminomethan-Puffer pH 8.8 in Gegenwart von 250 µl N-Methylpyrrolidon je Testansatz. Die Spaltungsgeschwindigkeiten wurden durch kontinuierliche

Messung des Extinktionsanstiegs bei 330 nm bestimmt.

Tabelle 3

| Aktivator | | µg/Test | relative Spaltungsgeschwindigkeit |
|---|---|---|---|
| Decanol | | 125 | 1.0 |
| Poly-L-Arg | (40000) | 20 | 3.6 |
| " | | 125 | 4.4 |
| Poly-DL-Lys | (37000) | 125 | 4.3 |
| Poly-D-Lys | (100000) | 125 | 4.5 |
| Poly-L-Lys | (14000) | 20 | 3.7 |
| " | | 125 | 4.8; 6.9 |
| " | (260000) | 125 | 4.55 |
| Poly-(L-$Lys_2$,L-Ala) | (32000) | 125 | 4.65 |
| Poly-L-Orn | (30000) | 20 | 3.7 |
| " | | 125 | 5.75; 6.9 |

In den Klammern sind die mittleren Molekulargewichte (Zahlenmittel) der Polyaminosäuren angegeben. Als Detergens wurde jeweils SDS in einer Menge von 10 µg pro Testansatz zugegeben.

Beispiel 4

Beschleunigung der enzymatischen Spaltung von Tosyl-L-alanin-3-hydroxy-5-phenylpyrrolester durch Leukozyten bei Zusatz von Polyaminosäuren in 0.1 M Tris-(hydroxymethyl)-aminomethan-Puffer pH 8.4 in Gegenwart von 250 µl N-Methylpyrrolidon je Testansatz. Die Spaltungsgeschwindigkeiten wurden durch kontinuierliche Messung des Extinktionsanstiegs bei 325 nm bestimmt.

Tabelle 4

| Aktivator | µg/Test | Detergens | µg/Test | relative Spaltungsgeschwindigkeit |
|---|---|---|---|---|
| Decanol | 125 | SDS | 10 | 1 |
| Poly-L-Arg (40000) | 125 | - | - | 4.8 |
| Poly-DL-Lys (37000) | 125 | - | - | 4.8 |
| Poly-L-Lys (14000) | 125 | - | - | 3.7 |
| Poly-L-Arg (40000) | 50 | SDS | 10 | 6.0 |
| " | 125 | SDS | 10 | 6.65 |
| Poly-L-Arg (40000) | 125 | BDTA | 20 | 4.05 |
| " | 125 | Heparinoid | 20 | 5.15 |
| Poly-DL-Lys (37000) | 125 | " | 20 | 6.55 |
| Poly-L-Arg (40000) | 50 | Zephirol | 20 | 6.35 |

SDS = Natriumdodecylsulfat
BDTA = Benzyl-dimethyl-tetradecyl-ammoniumchlorid

Zephirol wurde als ca. 50 %ige wäßrige Lösung eingesetzt.
In den Klammern sind die mittleren Molekulargewichte (Zahlenmittel) der Polymeren angegeben.

Allgemeine Arbeitsvorschrift zur Herstellung der N-Tosyl-L-alanylester:

Die Ester wurden jeweils durch Umsetzung von N-Tosyl-L-alanylchlorid mit den Phenolen in absolutem Methylethylketon oder absolutem Toluol in Gegenwart von gepulvertem Kaliumcarbonat hergestellt. Nach 6 bis 12-stündigem Rühren bei ca. 55° C waren zwischen 40 und 70 % des Phenols umgesetzt. Das Molverhältnis Phenol: $K_2CO_3$: Säurechlorid betrug zumeist 1:1,5:1,5. Der pH-Wert lag während der gesamten Reaktionszeit um 7. Zur Aufarbeitung wurde das Kaliumcarbonat bei 50° C abfiltriert und danach das Lösungsmittel im Vakuum abdestilliert. Die Reinigung erfolgte über Säulenchromatographie mit Kieselgel (Laufmittel Petrolether: Aceton = ca. 9:1) und anschließendes Umkristallisieren.

p-Tosyl-L-alanin
Literatur: E. Fischer u. W. Lipschitz, B. 48, 362 (1915).

83,7 g (0,93 Mol) L-Alanin werden in 465 ml ca. 2N-Natronlauge gelöst. Die Lösung wird bei 70-72° C portionsweise mit 186 g (0,976 Mol) p-Toluolsulfochlorid in 20 Minuten versetzt. Während der Zugabe des Sulfochlorids wird die Reaktionsmischung durch einen automatischen Titrator mit ca. 2N-Natronlauge bei pH 10 gehalten; dabei werden 560 ml 2N-Natronlauge verbraucht. Wenn sich der pH der Reaktionsmischung

nicht mehr ändert, kühlt man diese auf 15-5° C ab und stellt mit 37 %iger Salzsäure auf pH 3 ein. Das abgeschiedene Produkt wird abgesaugt, den feuchten Filterkuchen löst man aus 2350 ml Wasser um. Ausbeute: 185,5 g (82 % der Theorie) L-p-Tosylalanin vom m.p. 132-135° C.

p-Tosyl-L-alanylchlorid

158,1 g (0,65 Mol) p-Tosyl-L-alanin werden in 350 ml Thionylchlorid bei 40° C gerührt, bis eine klare Lösung entstanden ist. Dann destilliert man das überschüssige Thionylchlorid im Wasserstrahlvakuum ab. Den Kolbenrückstand nimmt man in 300 ml destilliertem Toluol auf. Man erhält eine klare, schwach gelbliche Lösung, die in 900 ml gerührtes Waschbenzin eingegossen wird. Das Säurechlorid fällt aus. Es wird am nächsten Tage abgesaugt, mit Leichtbenzin gewaschen und in einem Vakuumexsiccator über Calciumchlorid/Kaliumhydroxyd getrocknet.
Ausbeute: 155 g (91 % der Theorie) an fast farblosen Kristallen vom m.p. 81-83° C.

## Patentansprüche

**1.** Mittel zum Nachweis esterolytischer und/oder proteolytischer Enzyme, enthaltend
(a) einen Aminosäure- oder Peptidester eines Phenols als chromogenes Enzymsubstrat und
(b) eine die enzymatische Spaltung der Aminosäure- bzw. Peptidesterbindung von Komponente (a) beschleunigende Substanz,
dadurch gekennzeichnet, daß es als beschleunigende Substanz eine Polyaminosäure mit einem Molekulargewicht zwischen 1000 und 2 000 000 enthält.

**2.** Mittel nach Anspruch 1, dadurch gekennzeichnet, daß das Molekulargewicht der Polyaminosäure zwischen 5000 und 500 000 liegt.

**3.** Mittel nach Anspruch 1 oder 2, dadurch gekennzeichnet, daß die Polyaminosäure aus nur einer Aminosäure aufgebaut ist.

**4.** Mittel nach Anspruch 1 oder 2, dadurch gekennzeichnet, daß die Polyaminosäure aus zwei oder mehreren verschiedenen Aminosäuren mit statistischer Sequenz aufgebaut ist.

**5.** Mittel nach Anspruch 1 oder 2, dadurch gekennzeichnet, daß die Polyaminosäure aus zwei oder mehreren verschiedenen Aminosäuren mit sich wiederholender Aminosäuresequenz aufgebaut ist.

**6.** Mittel nach Anspruch 1 bis 5, dadurch gekennzeichnet, daß die Polyaminosäure aus Aminosäuren der allgemeinen Formel

$$H_2N - \underset{\displaystyle R}{\underset{|}{CH}} - COOH$$

aufgebaut ist, in welcher R für Wasserstoff oder einen gegebenenfalls verzweigten Alkyl-, Cycloalkyl- oder Aralkylrest mit 1 bis 15 C-Atomen, bevorzugt 1 bis 9 C-Atomen steht, welcher gegebenenfalls durch eine oder 2 Hydroxy-, Mercapto-, Carboxyl -, Amino- oder Guanidinogruppen substituiert ist.

**7.** Mittel nach Anspruch 1 bis 6, dadurch gekennzeichnet, daß 5 bis 100 Mol-%, bevorzugt 10 bis 100 Mol-%, der Aminosäurebausteine der Polyaminosäure eine basische Gruppe tragen.

**8.** Mittel nach Anspruch 7, dadurch gekennzeichnet, daß es sich bei den basischen Gruppen um Amino- und/oder Guanidinogruppen handelt.

**9.** Mittel nach Anspruch 1 bis 8, dadurch gekennzeichnet, daß die Reagenzien in einem inerten Trägermaterial, vorzugsweise in Form eines Teststreifens, inkorporiert sind.

**10.** Verfahren zum Nachweis von esterolytischen und/oder proteolytischen Enzymen in flüssigen Proben,

insbesondere Körperflüssigkeiten, dadurch gekennzeichnet, daß man die Probe mit einem Mittel nach Anspruch 1 bis 9 in Kontakt bringt und die auftretende Farbreaktion bestimmt.

## Claims

1. Agent for the detection of esterolytic and/or proteolytic enzymes, containing
   a) an amino acid ester or peptide ester of a phenol, as the chromogenic enzyme substrate, and
   b) a substance which accelerates the enzymatic cleavage of the amino acid ester or peptide ester bond of component (a),

   characterised in that it contains a polyamino acid with a molecular weight of between 1,000 and 2,000,000 as the accelerating substance.

2. Agent according to Claim 1, characterised in that the molecular weight of the polyamino acid is between 5,000 and 500,000.

3. Agent according to Claim 1 or 2, characterised in that the polyamino acid is built up from only one amino acid.

4. Agent according to Claim 1 or 2, characterised in that the polyamino acid is built up from two or more different amino acids in random sequence.

5. Agent according to Claim 1 or 2, characterised in that the polyamino acid is built up from two or more different amino acids with a recurring amino acid sequence.

6. Agent according to Claim 1 to 5, characterised in that the polyamino acid is built up from amino acids of the general formula

   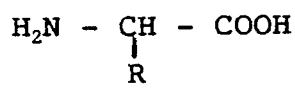

   in which R represents hydrogen or an optionally branched alkyl, cycloalkyl or aralkyl radical which has 1 to 15 C atoms, preferably 1 to 9 C atoms, and is optionally substituted by 1 or 2 hydroxyl, mercapto, carboxyl, amino or guanidino groups.

7. Agent according to Claims 1 to 6, characterised in that 5 to 100 mole%, preferably 10 to 100 mole%, of the amino acid units of the polyamino acid carry a basic group.

8. Agent according to Claim 7, characterised in that the basic groups are amino and/or guanidino groups.

9. Agent according to Claims 1 to 8, characterised in that the reagents are incorporated in an inert carrier, preferably in the form of a test strip.

10. Process for the detection of esterolytic and/or proteolytic enzymes in liquid samples, in particular body fluids, characterised in that the sample is brought into contact with an agent according to Claims 1 to 9 and the colour reaction which occurs is determined.

## Revendications

1. Composition pour la détection d'enzymes estérolytiques et/ou protéolytiques, contenant
   (a) un ester d'amino-acide ou de peptide d'un phénol comme substrat d'enzyme chromogène et
   (b) une substance accélérant la siccion enzymatique de la liaison ester d'amino-acide ou de peptide du composant (a),

   caractérisée en ce qu'elle contient comme substance accélératrice un polyamino-acide de poids moléculaire compris entre 1000 et 2 000 000.

2. Composition suivant la revendication 1, caractérisée en ce que le poids moléculaire du polyamino-acide se situe entre 500 et 500 000.

3. Composition suivant la revendication 1 ou 2, caractérisée en ce que le polyamino-acide n'est constitué que d'un amino-acide.

4. Composition suivant la revendication 1 ou 2, caractérisée en ce que le polyamino-acide est constitué de deux ou plus de deux amino-acides différents, à séquence statistique.

5. Composition suivant la revendication 1 ou 2, caractérisée en ce que le polyamino-acide est constitué de deux ou plus de deux amino-acides différents à séquence répétitive des amino-acides.

6. Composition suivant les revendications 1 à 5, caractérisée en ce que le polyamino-acide est constitué d'amino-acides de formule générale

$$H_2N - \underset{\displaystyle R}{\underset{|}{CH}} - COOH$$

dans laquelle R représente l'hydrogène ou un reste alkyle éventuellement ramifié, cycloalkyle ou aralkyle ayant 1 à 15 atomes de carbone, de préférence 1 à 9 atomes de carbone, qui est substitué le cas échéant par un ou deux groupes hydroxy, mercapto, carboxyle, amino ou guanidino.

7. Composition suivant les revendications 1 à 6, caractérisée en ce que 5 à 100 moles %, de préférence 10 à 100 moles %, des motifs amino-acides du polyamino-acide portent un groupe basique.

8. Composition suivant la revendication 7, caractérisée en ce que les groupes basiques sont des groupes amino et/ou guanidino.

9. Composition suivant les revendications 1 à 8, caractérisée en ce que les réactifs sont incorporés dans un support inerte, de préférence sous forme d'une bandelette réactive.

10. Méthode de détection d'enzymes estérolytiques et/ou protéolytiques dans des échantillons liquides, notamment dans des liquides corporels, caractérisée en ce qu'on met l'échantillon en contact avec une composition suivant les revendications 1 à 9 et on évalue la réaction colorée qui se produit.